Europäisches Patentamt

European Patent Office

Office européen des brevets

(19)

(11) Publication number: **0 239 244 B1**

# EUROPEAN PATENT SPECIFICATION

(12)

(45) Date of publication of patent specification: **18.09.91**  (51) Int. Cl.⁵: **A61M 5/00**

(21) Application number: **87301577.0**

(22) Date of filing: **24.02.87**

(54) Subcutaneous injection set.

(30) Priority: **25.02.86 US 834173**

(43) Date of publication of application:
**30.09.87 Bulletin 87/40**

(45) Publication of the grant of the patent:
**18.09.91 Bulletin 91/38**

(84) Designated Contracting States:
**DE FR GB IT NL SE**

(56) References cited:
**DD-A- 67 214        GB-A- 1 240 312**
**GB-A- 2 162 752      US-A- 4 000 739**
**US-A- 4 235 234      US-A- 4 393 873**
**US-A- 4 464 178      US-A- 4 531 937**

(73) Proprietor: **PACESETTER INFUSION LTD.**
**12884 Bradley Avenue**
**Sylmar California 91342(US)**

(72) Inventor: **Lord, Peter C.**
**25505 Old Course Way**
**Valencia California 91355(US)**
Inventor: **Konopka, April A.**
**510 South San Marcos Road**
**San Dimas California 91773(US)**

(74) Representative: **Rees, David Christopher et al**
**Kilburn & Strode 30 John Street**
**London WC1N 2DD(GB)**

## Description

The present invention relates generally to injection devices for use with an external infusion system in which a desired fluid is subcutaneously delivered to a patient. The invention may be particularly applicable to a disposable subcutaneous injection set in which for example a soft cannula or flexible needle is inserted approximately normal to the skin to a desired subcutaneous injection level and thereafter delivers the fluid with a minimum of discomfort and bother to the patient.

In general, whenever a prescribed fluid is to be delivered subcutaneously to a patient from an external source, a passageway such as a hollow needle or other type of cannula or catheter device must first be inserted through the skin of the patient in order to provide a passageway or channel through which the fluid may pass from its source outside the patient to the desired subcutaneous location under the skin of the patient. Once this passageway has been installed, any suitable infusion device or system in conjunction with an appropriate catheter connecting the external source of fluid with the passageway leading to the subcutaneous delivery point may be used to deliver the fluid to the patient at an appropriate delivery rate.

Unfortunately, several problems attendant to infusing fluids into the patient as described above are usually encountered. In the first place, it is often uncomfortable, painful, and inconvenient to have a hollow needle or equivalent device piercing the patient's skin for prolonged periods of time. In addition, securing means must be used to keep the needle or equivalent device from moving from or within the injection site as the patient moves about. Such securing means are often quite bulky and bothersome to the patient, as well as being unsightly. There is also a continual risk of infection or inflammation at the skin puncture site. Moreover, the injection system must be primed prior to use. This involves removing all air or other gas from the connecting catheter and needle in order to allow a known quantity of fluid to be safely dispensed.

A number of solutions attempting to address these problems have been offered. For example, it is known in the art to insert a soft cannula into the patient rather than a stiff, hard needle or equivalent device. Such a device is shown in U.S. Patent No. 4,531,937, Yates and this document is used for the delineation of Claim 1 of the present application. A soft cannula is much more comfortable to the patient than such rigid devices. Insertion is accomplished using a removable stiff needle or stylet that passes tightly through a lumen of soft cannula. A sharp tip of the needle extends from the end of the cannula when the needle is fully inserted therein. The sharp tip of the needle is inserted into the patient at the desired injection point, with the soft cannula following the needle and thereby being inserted therewith. Once both the needle and soft cannula are inserted, the needle is withdrawn from the cannula, leaving the soft cannula in the patient, so providing a more flexible and comfortable passageway through which fluid may be delivered to the patient at the desired subcutaneous site.

In this and other similar devices, however, removal of the needle from the soft cannula leaves an opening through which fluid may leak. While such an opening may be plugged by locating a self-sealing septum in the cannula, the problem of some degree of fluid leakage remains, particularly in those drug infusion systems having fluid delivery pressures much greater than those pressures encountered in gravity-feed injection systems.

Another proposed solution to the foregoing problems is a hard needle injection set as shown in U.S. Patent No. 4, 235, 234, to Whitney, et. al. The injection set includes a locator pad having a sharp rigid needle protruding substantially at right angles to the bottom surface of the locator pad. The bottom surface of the locator pad typically has a pressure sensitive adhesive applied thereto. Thus the needle is inserted into the skin of the patient while the locator pad is pressed against the skin at the same time in substantially the same motion, thereby securing the needle at the desired location. The needle has a right angle bend within the locator pad, to allow a delivery tube to be connected to the needle in a direction substantially parallel to the skin. This eliminates tubes or needles that might otherwise protrude perpendicularly from the skin, which protrusions can be not only a source of constant irritation and frustration to the patient, but also an easy target for accidental bumping by or entanglement with the patient's external environment.

Despite the beneficial features available with teachings of the art such as Whitney, et al, some problems common to injection sets still persist. For example with hollow needle injection systems a common problem is for the relatively small diameter lumen of the needle to become clogged or other wise blocked or plugged, thereby preventing the free flow of fluid. This problem is aggravated in devices having a 90 degree bend in the needle. For this reason, most injection sets (unlike Whitney and equivalent devices) shy away from 90 degree bends in the delivery channel. Many of these devices utilise a "Y" shape, thereby minimising the angle or bend in which a blockage could occur.

Another significant problem that occurs whenever a hollow needle or equivalent device (e.g., a solid needle having a channel or groove along one side) is employed to puncture the skin is that of "body coring". Body coring occurs when a piece of body tissue is cored out of the body as the needle enters. This piece of cored-out tissue remains in the lumen or channel of the needle and prevents fluid from flowing through it. In many injection systems, such as that illustrated in Whitney, et. al., a body core can only be removed by removing the needle from the patient and flushing the system to force the cored tissue from the needle.. Unfortunately, upon reinsertion there is a substantial risk that body coring may occur again. In other injection systems, such as those typified by Yates, body coring can prevent the system from being primed in the proper manner.

In view of the above, it can be seen that a substantial need exists for an injection set that combines the advantageous features of the devices discussed above but without the disadvantages encountered by such devices, namely the difficulties associated with priming and sealing the devices, and the problems associated with blocking or plugging of the devices.

According to the invention, there is provided a subcutaneous injection set comprising a housing, a hollow needle protruding from the housing and means for connecting a source of fluid to the needle; the housing including a hub having a chamber therein, and self-sealing means for enclosing the chamber at the top of the hub; the needle comprising a soft or flexible cannula having a lumen which is in fluid communication with the chamber within the housing, and an insertion needle for inserting the protruding portion of the cannula into a subcutaneous layer of the patient, the insertion needle passing removably through the self-sealing means at the top of the hub, through the chamber and through the lumen of the soft cannula with a tight fit, the insertion needle having a sharpened tip extending beyond the length of the soft cannula when the insertion needle is fully inserted through the housing, the self-sealing means sealing the top of the hub when the insertion needle is withdrawn therefrom, characterised in that the housing comprises a holding pad with a substantially flat base for placement on the skin of a patient at an injection site, means for removably securing the holding pad to the skin of a patient; and in which the hub is mounted on top of the holding pad and the soft cannula extends from the chamber through the hub and the holding pad.

Preferably, the insertion needle includes means for gripping the end opposite the tip, the means for gripping being accessible from the upper side of the housing the upper side being on the opposite side of the housing from the flat surface, whereby the sharpened tip of the insertion needle punctures the skin and enters a subcutaneous fat layer of the patient as the flat surface of the housing is pushed against the skin of the patient. The end of the cannula is carried into the subcutaneous fat layer with the insertion needle, and the insertion needle is then retracted from the lumen of the cannula once the end of the cannula has been inserted into the subcutaneous fat layer. Preferably, the self-sealing means comprises a septum made from a pierceable resilient resealable substance which closes the top of the chamber, and through which the insertion needle may be removably inserted.

Preferably, the injection set includes compression means for subjecting the self-sealing septum affixed to the upper side of the housing to constant compressive forces, whereby any hole made in the septum by the insertion needle is tightly closed by the compressive forces once the insertion needle is removed. Preferably, the compressive forces include both radial compressive forces and shearing compressive forces acting on the septum.

Preferably, the compression means includes a cap which is affixed to the upper side of the housing over the septum in a way which exerts a compressive force on the septum, the cap having a small hole through which the insertion needle may be inserted. The compression may also be achieved by sizing the self-sealing septum somewhat larger than the opening in the upper side of the housing into which the septum is inserted.

The septum may comprise a plurality of septum layers each of which may be subjected to compressive force. Successive ones of the septum layers may be made of at least two different materials, each of the different materials having different compressive characteristics. The different materials may include bytyl and silicone.

In a preferred embodiment, the septum comprises: a first layer of vulcanised silicone; a second layer of vulcanised silicone; and a layer of silicone gel disposed between the first and second layers of vulcanised silicone, the silicone gel providing a complete seal of any residual openings in the first and second layers of vulcanised silicone when the insertion needle is withdrawn therefrom.

Fluid may be supplied to the chamber via a standard infusion set having an angled needle inserted through the septum following removal of the insertion needle. Preferably, however, there is a fluid inlet to the chamber through a side of the housing adjacent to and between the base and upper side of the housing. In one preferred arrangement the fluid inlet includes a flexible tube in fluid communication with the fluid chamber within the housing, the flexible tube extending from the side wall of the housing at an angle which is substantially parallel to the flat base. In an alternative arrangement, the fluid inlet is closed by a second self-sealing septum through which an infusion needle may be removably inserted. The second septum may comprise at least one septum layer made from a pierceable resilient, resealable substance.

The cannula may be made from any suitable material, examples of which are teflon (or polytetrafluorethylene) and hydrogel material. Preferably, the cannula is tapered, to enhance insertion.

The apparatus preferably includes means for detachably securing the flat base of the housing to the skin of the patient, comprising an adhesive layer placed on the flat base. Thus, one side of the adhesive layer would be attached to the flat base of the housing with the other side of the adhesive layer sticking to the skin of the patient as soon as contact is made between the adhesive layer and the skin.

Preferably, the adhesive layer includes thereon an antimicrobial substance on the skin side; the antimicrobial substance reduces the risk of infection as the injection set is used by the patient. Preferably, the skin side of the adhesive layer is covered with a disposable covering which may be manually removed when the injection set is ready to be affixed to the skin of the patient, the disposable covering having a hole through which the soft cannula and insertion needle pass, the disposable covering also having a slit which passes from the first hole to the outer edge of the disposable covering. The disposable cover may also have a finger tab which projects out from the edge adjacent the slit, whereby the disposable covering may be removed from the adhesive by pulling the finger tab away from the base of the housing with a circular motion which does not require lifting or sliding the disposable covering over the soft cannula or insertion needle.

The housing itself preferably comprises a domed base portion having a flat surface which comprises a flat base and a centrally located aperture leading through the base; and a central hub portion containing the chamber. The central hub portion is preferably mounted on the side of the domed base portion opposite the flat surface, and preferably has an aperture leading from the chamber to the centrally located aperture in the domed base portion. The central hub portion preferably includes the upper side in which the first self-sealing septum is located.

Preferably, the injection set further includes means for priming the injection set when the chamber is connected to a source of fluid prior to insertion of the insertion needle and the soft cannula into the skin of a patient, the priming means comprising a passage between the tip of the insertion needle and a section of the insertion needle within the chamber, the passage being in fluid communication with the chamber, whereby all air or other gases within the fluid chamber may escape through the passage when the tip of the insertion needle is held so as to point upwards as fluid is introduced under pressure into the chamber.

The passage may comprise a bore located within the insertion needle and an aperture from the exterior of the insertion needle to the bore, the aperture being located in the chamber at a position near the base of the housing when the insertion needle is fully inserted through the housing. Alternatively, the passage may comprise a longitudinal groove disposed along the side of the insertion needle.

The present invention therefore teaches a disposable injection set that offers a soft or flexible cannula that in the preferred embodiment is inserted essentially perpendicularly into the skin of a patient and held at its insertion location with a low profile holding pad or housing. The low profile holding pad is affixed to the skin of the patient, preferably with an antimicrobial pressure sensitive adhesive applied to a bottom surface thereof. The soft cannula protrudes from the bottom surface of the holding pad to a desired subcutaneous delivery level. An external source delivers fluid to the injection set via a catheter or tube that connects with the low profile holding pad at an angle substantially parallel to the skin of the patient, thereby minimising any noticeable protrusion and the attendant nuisance or hindrance to the patient.

A fluid chamber within the low profile holding pad or housing is used to connect the soft cannula, which is secured within the holding pad, with the delivery tube. This connection is accomplished in a way that eliminates any sharp bends or narrow openings particularly susceptible to clogging. Preferably, a rigid insertion needle included with the injection set is initially inserted through a self-sealing septum wall contained in the housing, and this needle tightly fits within the lumen in the soft or flexible cannula. The insertion needle has a sharp tip which extends beyond the end of the cannula when the insertion needle is fully inserted into the injection set. This sharp tip is used to puncture the skin of the patient and thereafter, the insertion needle provides the support for inserting the soft cannula subcutaneously into the patient. Once the soft cannula and insertion needle are inserted into the patient, and the bottom surface of the holding pad is pressed against and affixed to the skin of the patient, the insertion needle may be removed from the injection set, thereby leaving the soft cannula inserted into the patient. Preferably, a specially constructed self-sealing septum in the housing, through which the insertion needle is inserted, assures that the fluid does not leak out of the housing once the needle has been withdrawn.

A notable feature of the present invention is the ease with which the soft or flexible cannula may be inserted and secured to the skin of the patient. Additionally, there need be no concern as to whether body cored tissue will plug or stop delivery of the fluid, or other wise interfere with priming of the system, because priming occurs before insertion and therefore before any body coring could occur. Following insertion, any cored tissue will remain in the insertion needle, and when the needle is removed, it will carry any cored tissue with it out of the device. The present invention also has the ability to allow the injection set to be primed prior to insertion of the soft cannula into the patient.

Since the holding pad preferably includes a layer of pressure sensitive adhesive on its bottom surface, which, in the preferred embodiment includes an anti-microbial substance to minimise the risk of infection or inflammation at the skin puncture point, only minimal preparation needs to be done to the patient's skin at the injection site prior to insertion of the insertion needle and application of the holding pad onto the skin.

Another feature of the present invention is the free flow sub-cutaneous delivery channel that is provided once the injection set has been put in place, since this delivery channel has the capacity to deliver larger volumes of fluid than have previously been possible.

Finally, the present invention preferably, has a specially configured self-sealing septum which seals the device once the insertion needle has been withdrawn, even when the septum is subjected to internal fluid pressures of the magnitude commonly developed with modern drug infusion devices.

According to another aspect of the invention, there may be provided a subcutaneous injection set comprising: a housing having a fluid chamber therein; means for detachably securing a first side of said housing to the skin of a patient; a soft cannula having a lumen therethrough to allow fluid communication with said fluid chamber through said lumen, said soft cannula protruding from said first side of said housing; first self-sealing means for sealing a second side of said housing; means for receiving a desired fluid into said fluid chamber; and an insertion needle for removable insertion through said first self-sealing means in said second side of said housing, said insertion needle passing through said fluid chamber and through said lumen of said soft cannula, said insertion needle having an outside diameter size which tightly fits and passes through the lumen of said soft cannula, a sharpened tip of said insertion needle extending beyond the length of said soft cannula when said insertion needle is fully inserted through said housing, said first self-sealing means for sealing said second side of said housing when said insertion needle is withdrawn therefrom.

According to another aspect of the invention, there may be provided an injection set for injecting a desired fluid from a source of fluid to a subcutaneous delivery location under the skin of a patient, said injection set comprising: a housing having a substantially flat surface on one side thereof, said flat surface being adapted for placement against the skin of the patient; a flexible cannula having a first end affixed within said housing and a second end which protrudes from said flat surface, said cannula having a lumen therein extending between said first and second ends; a delivery tube having a first end affixed to said housing, said delivery tube being substantially parallel to said flat surface as it approaches and is attached to said housing, a second end of said delivery tube being connectable to said source of fluid; fluid connection means within said housing for connecting the first end of said delivery tube to the first end of said flexible cannula; and cannula insertion means for inserting the protruding body portion of the flexible cannula into the subcutaneous fat layer of the skin of the patient as the flat surface of the housing is placed against the skin of the patient.

In a broad sense, the present invention may be considered to comprise a subcutaneous injection device comprising a locator pad for placement against the skin of a patient; a flexible cannula having a distal tip protruding from a bottom surface of said locator pad, a proximal tip of said flexible cannula being secured to said locator pad; and fluid inlet means in fluid communication with the proximal tip of said flexible cannula, said fluid inlet means being in a plane which is substantially parallel to the bottom surface of said locator pad.

A preferred form of the invention may be considered to comprise an injection set for injecting a desired fluid from a source of fluid to a delivery location under the skin and in the subcutaneous fat layer of a patient, comprising: a domed base portion having a flat surface on one side thereof for placement against the skin of the patient, said domed base portion having an aperture therethrough; a central hub portion mounted on the side of said domed base portion opposite said flat surface, said central hub portion having a fluid chamber therein said central hub also having a passageway leading from said fluid chamber to the side of said central hub portion mounted on said domed base portion, said passageway being in communication with said aperture in said domed base portion, said central hub portion having an opening on the side of said central hub portion opposite said passageway which opening is in communication with said fluid chamber; a soft cannula having a first end in communication with said fluid chamber, said soft cannula extending through said passageway in said central hub portion and through said aperture in said domed base portion with a second end of said soft cannula thereby extending from said flat surface of said domed base portion, said soft cannula having a lumen therethrough; a self-sealing septum portion for sealing said opening in said central hub portion; an insertion needle for removable insertion into said self-sealing septum portion, through said fluid chamber in said central hub portion and said lumen in said soft cannula, said insertion needle having a sharpened tip thereon at the end coming out of said second end of said cannula said sharpened tip extending from said second end of said soft cannula when said insertion needle is fully inserted, said self-sealing septum portion sealing when said insertion needle is withdrawn; said means for receiving fluid from said source into said fluid chamber.

The injection set according to the invention may be used for a method of injecting a fluid subcutaneously into a patient, said method comprising the steps of: placing an insertion needle through a self-sealing top portion of said locator pad, through said chamber, and through a lumen of said soft cannula such that a sharpened tip of said insertion needle extends beyond a distal tip of said soft cannula, said insertion needle having air passage means for allowing the passage of air from said chamber to a point on said insertion needle beyond a distal tip of said soft cannula; connecting said inlet delivery tube to a source of said fluid; delivering said fluid from the source of said fluid, through said inlet delivery tube, into said chamber, and through said air passage means, so as to expel all of the air from said inlet delivery tube, chamber, and air passage means; inserting the sharpened tip of said insertion needle into the skin of the patient; positioning the bottom surface of the locator pad against the skin of the patient while maintaining the insertion of said insertion needle in the skin of the patient; retracting said insertion needle from said lumen of said soft cannula, said chamber, and said self-sealing tip portion of said locator pad; and delivering fluid from said source fluid, through said inlet delivery tube, through said chamber, and through said soft cannula to a subcutaneous delivery point in the patient.

The invention also extends to a method of making a subcutaneous injection set, comprising: providing a locator pad having a chamber therein, said chamber having an open top accessible from a top surface of said locator pad, said locator pad also having a bottom surface, said bottom surface having disposed therein a first channel which passes between said bottom surface and a bottom of said chamber; sealably affixing a flexible cannula in said first channel, a portion of the length of said cannula protruding normally from said bottom surface, said cannula having a lumen therethrough;

sealably locating a septum in the top surface of said locator pad so as to seal the open top of said chamber, said septum being under a compressive force as it is affixed to said locator pad; and inserting an insertion needle through said septum, said chamber, and said lumen of said flexible cannula, said insertion needle having a tip which extends beyond the protruding flexible cannula when said insertion needle is fully inserted, said insertion needle having air passage means therein for allowing air to pass from said chamber to the tip of said needle.

The invention may be carried into practice in various ways and some embodiments will now be described by way of example with reference to the accompanying drawings, in which:

Figure 1 is a perspective view of a soft cannula subcutaneous injection set in accordance with the present invention shown as it would appear prior to being applied to a patient;

Figure 2 is a sectional view of the injection set of Figure 1 when inserted into the skin of the patient and before the insertion needle has been withdrawn;

Figure 3a is a sectional view taken along the line 3A-3A of Figure 2 for a hollow insertion needle embodiment of the invention;

Figure 3B is a sectional view taken along the line 3B-3B of Figure 2 to show the aperture in the hollow insertion needle embodiment of the invention;

Figure 3C is an analogous view of the view taken along the line 3A-3A and shown in Figure 3A, but for a grooved insertion needle embodiment of the invention;

Figure 4 is a sectional view of the injection set of Figure 1 when inserted into the skin of the patient and after the insertion needle has been withdrawn;

Figure 5A is an enlarged view of a multi-layer septum having a needle inserted therethrough;

Figure 5B is an enlarged view of a multi-layer septum as in Figure 5A with the needle removed therefrom;

Figure 6 is a perspective view of the injection set of Figure 1 showing how a protective covering is removed from pressure-sensitive adhesive on the bottom side of the holding pad, a needle guard installed over the needle and cannula, and an alternative shape for the handle of the needle;

Figure 7A is an enlarged view of an alternative embodiment using a silicone gel system septum having a needle inserted therethrough;

Figure 7B is an enlarged view of the silicone gel system septum as in Figure 7A with the needle removed therefrom;

Figure 8 is a sectional view of an alternative embodiment top port button injection set; and

Figure 9 is a sectional view of an alternative embodiment side port button injection set.

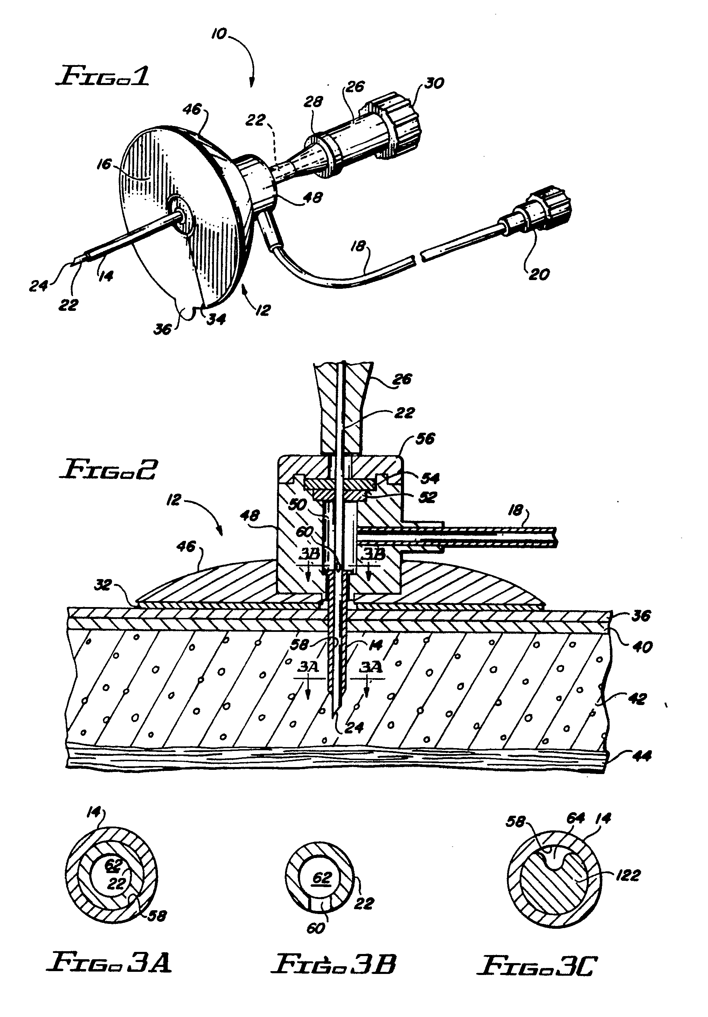

Referring to Figure 1, the injection set 10 includes a holding pad 12 having a soft cannula 14 protruding from its bottom surface 16 at an angle of between 15 and 90 degrees, in this case approximately perpendicularly. A delivery tube 18 is connected at one end to the holding pad 12 at an angle substantially parallel to the bottom surface 16. The other end of the delivery tube 18 is fitted with a conventional female connector 20 which may be readily connected or coupled to a suitable source of fluid (not shown).

Prior to insertion into the patient, the soft cannula 14 has a hard insertion needle 22 inserted therein. The insertion needle 22 has a sharp tip 24 which extends beyond the end of the soft cannula 14 when the insertion needle 22 is fully inserted into the holding pad 12. The insertion needle 22 further includes a handle 26 or other means for allowing the insertion needle 22 to be firmly gripped by the fingers of a person who is inserting or removing the injection set 10. The handle 26 shown in Figure 1 includes a lower rim 28 and an end cap 30 to facilitate gripping the handle 26. The lower rim 28 may be gripped between two fingers while a thumb may be placed against the top of the end cap 30, thereby providing a means for applying both pushing and pulling forces on the insertion needle 22. Other types of handles could, of course, be used to facilitate gripping of the insertion needle 22. For example, an alternative type of handle 80 is shown in Figure 6.

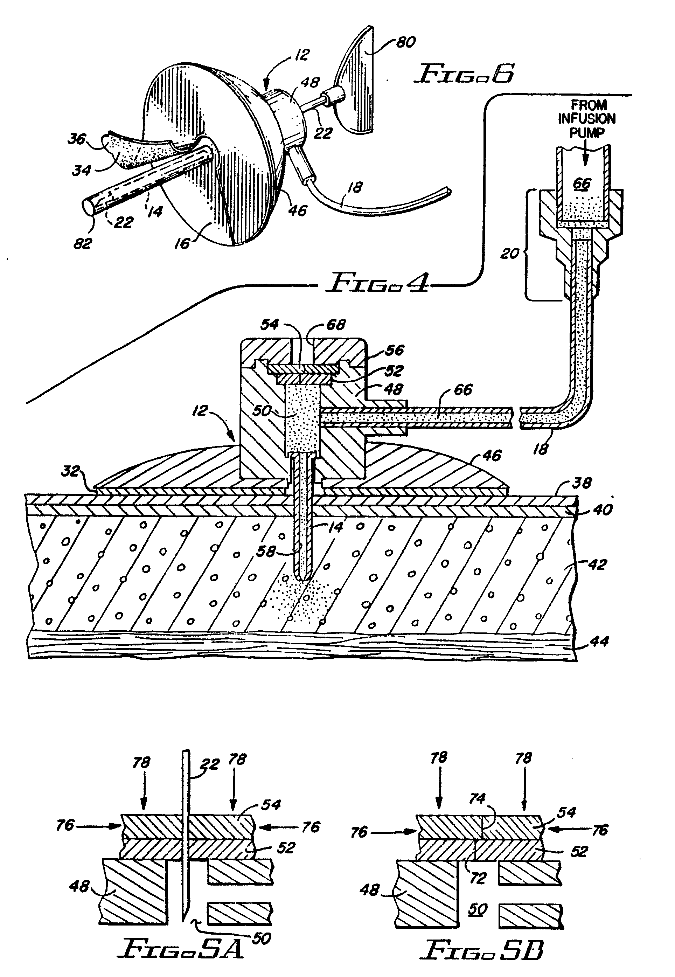

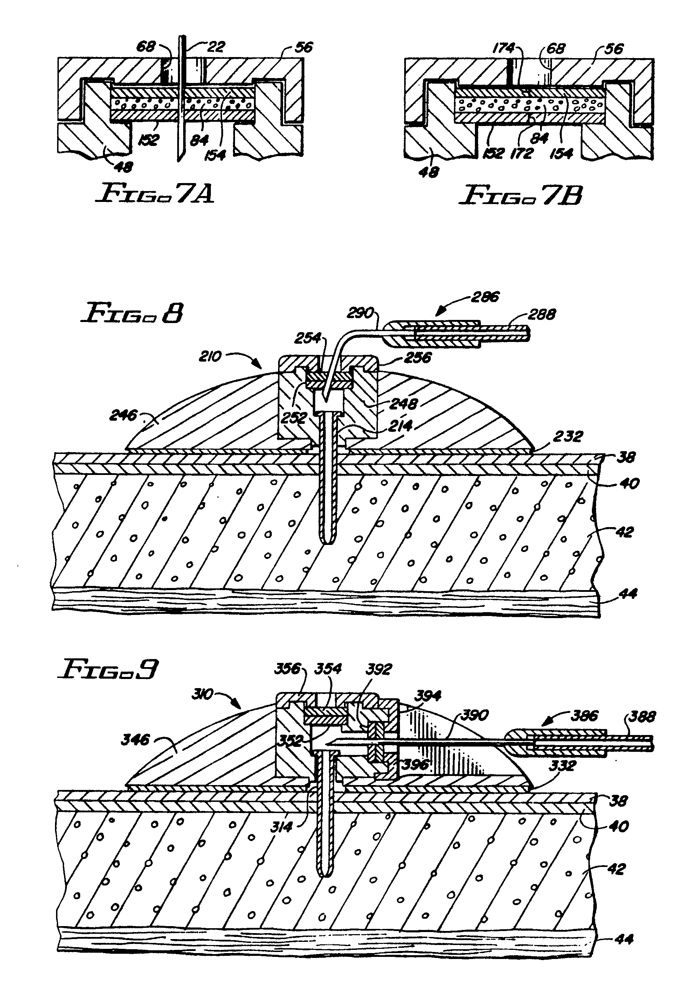

The bottom surface 16 of the holding pad 12 is coated with a layer of pressure-sensitive adhesive 32 (best shown in Figures 2 and 4). This layer 32 is covered with a protective paper backing 34 having a pull tab 36. The manner in which the protective paper backing 34 is removed from the bottom surface 16 will be explained more fully below in conjunction with Figure 6.

Referring next to Figure 2, a sectional view of the injection set 10 taken generally through its centre is shown after the injection set 10 has been inserted into the skin of a patient, but prior to removal of the insertion needle 22. The skin of the patient is comprised of several layers of body tissue as shown in Figure 2. An epidermis layer 38 comprises the outer layer of skin. Under the epidermis layer 38 and the dermis layer 40 is a subcutaneous fat layer 42. Muscle tissue 44 generally lies beneath the subcutaneous fat layer 42. In accordance with the teachings of the present invention, the soft cannula 14 is inserted through the layers of skin 38 and 40 and into the subcutaneous fat layer 42. It is desirable that the cannula 14 should not protrude into the muscle tissue 44, since the muscle tissue 44 absorbs insulin at a different rate than does the subcutaneous fat layer 42.

The holding pad 12 includes a domed base portion 46 and a central hub portion 48. A fluid chamber 50 is centrally located within the central hub portion 48. Both the soft cannula 14 and the delivery tube 18 are in fluid communication with the fluid chamber 50.

For the embodiment shown in Figure 2, a first septum layer 52 and a second septum layer 54 cover or enclose the top of the fluid chamber 50. A copy 56, suitably bonded to the top portion of the central hub 48, securely holds the septum layers 52, 54 in their desired location, as explained more fully below. The insertion needle 22 is inserted through the septum layers 52, 54 and through a lumen 58 of the soft cannula 14. Fluid communication means are provided within the insertion needle 22 to allow fluid, and hence air or other gases, to flow readily between an aperture or opening 60 of the insertion needle 22 (which is positioned within the chamber 50 when the insertion needle 22 is fully inserted into the injection set 10) and the insertion needle tip 24. As seen in the sectional views of Figures 3A and 3B, this fluid communication means may be achieved with at least one of two different embodiments. In the embodiment shown in Figure 3A, the insertion needle 22 is hollow having a bore 62 through its centre. In the alternative embodiment shown in Figure 3B, an insertion needle 122 is shown which is solid and has a groove or channel 64 along one side. In either embodiment, a tight fit within the lumen 58 of the soft cannula 14 is achieved. It should also be noted that the end of the soft cannula 14 is slightly tapered to enhance its insertion. The needle bore 62, in conjunction with the hole or aperture in the insertion needle 22 positioned within the fluid chamber 50 (for the embodiment of Figure 3A), or the groove or channel 64 of the insertion needle 122 which would terminate within the fluid chamber 50 (for the embodiment of Figure 3B), provides the fluid communication means between the fluid chamber 50 and the needle tip 24.

The fluid communication means provided by the bore 62 or the groove 64 allows the injection set 10 to be easily primed prior to insertion into the patient. All that needs to be done to prime the injection set 10 is to point the tip 24 of the insertion needle 22 in an upwards direction at the same time that the fluid to be injected is inserted into the end of the delivery tube 18 having the connector 20. As the fluid passes through the delivery tube 18 into the fluid chamber 50, all of the air that was previously inside the delivery tube 18 and the fluid chamber 50 will escape through the bore 62 (or groove 64) of the needle 22 (122).

Since the fluid chamber 50 is very small, typically less than one-half unit in volume, the removal of all the air by this operation would probably take place irrespective of the positioning of the tip 24, however, this described positioning is recommended. When all of the air has thus been removed, and the system is primed, the fluid itself will begin to leave from the tip 24 of the insertion needle 22, thereby signalling to the person using the device that the desired priming has been accomplished. Advantageously, in the preferred embodiment, the material from which the delivery tube 18 and the central hub 48 of the holding pad 12 are made is transparent, and thereby provides a means for visually determining whether all air bubbles have been removed from the delivery tube 18 and the fluid chamber 50.

With all of the air thus removed from the system, the injection set 10 may then be applied to the patient in the same manner as any needle would be inserted into a patient by a nurse, or doctor, or by the patient himself or herself. As stated above, the amount of fluid contained within the tube 18 and fluid chamber 50 of the primed injection set is not large, typically less than one-half unit. In any event, it is a known amount that the patient and/or doctor can use, as needed, to determine the amount of fluid that has been delivered to the patient over a period of time.

Referring next to Figure 4, a sectional view of the injection set 10 is shown as it appears when attached to a patient after the insertion needle 22 (shown in Figure 2) has been removed. Figure 4 also shows a desired fluid 66 (represented as small dots) delivered from an infusion pump (not shown) or similar delivery system flowing through the delivery tube 18, into the fluid chamber 50, and through the lumen 58 of the soft cannula 14 so as to be dispersed at the desired delivery point within the subcutaneous fat layer 42 of the patient. The inner diameter of the lumen 14, typically approximately 24 gauge and the smallest opening associated with the delivery system, allows appropriate amounts of the fluid 66 to be delivered under control of the infusion pump (not shown) without excessive flow restrictions.

The injection set 10 as shown and described herein has two septum layers 52, 54, however, the teachings of the present invention apply equally to devices having one or more septum layers. In fact, the additional layers may be used to provide even greater assurance of an effective seal. As shown in Figure 4, the septum layers 52, 54 reseal once the insertion needle 22 (figure 2) has been withdrawn, thereby preventing the fluid 66 from leaking out of the chamber 50 through an aperture 68 in the end cap 56. Because the fluid 66 is typically delivered from the infusion pump (not shown) under a substantial pressure (at least relative to the minimal pressures that are typically associated with gravity-flow fluid delivery systems), it is critically important that the septum layers 52,54 provide an effective seal for preventing the fluid 66 from escaping or leaking out of the fluid chamber 50. In order to ensure a tight seal at the septum layers 52,54, these septum layers 52,54 are prestressed with compressive forces as illustrated in the partial sectional views of Figures 5A and 5B.

Referring firstly to Figure 5A, a partial sectional view of the fluid chamber 50 and septum layers 52,54, having the insertion needle 22 inserted therethrough, is shown. Insertion of the insertion needle 22 through the septum layers 52,54 necessarily causes the cutting or making of openings 72,74 through the septum layers 52,54 respectively. In a conventional septum, the resilient nature of the septum material is relied upon to close the openings 72,74 once the piercing insertion needle 22 has been removed. In order to ensure that this sealing or closing occurs, compressive forces are applied to the septum layers 52,54 in at least two directions.

For example, as represented by the horizontal arrows 76 the septum layers 52,54 are compressed radially inwardly towards their centres. Similarly, as represented by the downward pointing arrows 78, a shearing compressive force is applied to the upper side of the septum layers 54, 52. In the preferred embodiment, adjacent septum layers such as 52,54 are made of different materials. Also, the effective forces on each of the septum layers 52,54 will necessarily differ. The net effect of typical compressive forces applied to the septum layers 52,54 is illustrated in Figure 5B, wherein it is seen that any residual openings 72,74 through the septum layers 52,54 respectively, will not align, thereby assuring that an effective seal is achieved.

The horizontal compressive forces 76 are achieved simply by sizing the septum layers 52,54 to be slightly larger than the openings into which they are inserted. The vertical compressive forces 78 are achieved by applying the end cap 56 over the septum layers as shown in Figures 2 and 4 in order to apply a constant downward pressure or force. In the embodiment shown, the septum layer 52 may be of butyl, a substance known to be compatible with insulin and other fluids likely to be injected through the injection set of the present invention. The septum layer 54, on the other hand, may be of silicon. Because silicon and butyl have somewhat different properties, especially in response to the compressive forces that are applied, the use of different materials in these respective septum layers further assures that an appropriate seal is achieved. As stated above, while two septum layers are shown in the figures, any number of septum layers subjected to appropriate compressive forces could be used as described.

The method of making an injection set 10 according to the present invention will now be described. First, the domed base 46 and the central hub 48 of the holding pad 12 are moulded using appropriate known moulding techniques and tools. While the sectional views presented herein suggest that the central hub 48 is a separate part from the domed base 46, this is not essential. In practice, it may be easier to mould the central hub 48 and domed base 46 as an integral part. In the preferred embodiment, the materials from which the domed base 46 and central hub 48 are made are a rigid material such as rigid PVC for the central hub 48, and a flexible material such as flexible PVC for the domed base 46. These materials will typically cure so as to be clear or transparent, thereby allowing visual indication that all of the air has been removed from the system when the system is primed.

The moulding process would include means for leaving an aperture or channel in the bottom of the central hub 48 and through the centre of the domed base 46 through which the soft cannula 14 may be attached. A second aperture through which the delivery tube 18 may be attached is located in the side of the central hub 48. The soft cannula 14 may be made of a teflon tube of an appropriate diameter and thickness. The soft cannula 14 is inserted and swedged or heat sealed to the hub 48 so as to be in fluid communication with the fluid chamber 50. Similarly, the delivery tube 18 may be sealably bonded with solvent to the hub 48 so as to be in fluid communication with the chamber 50.

An alternative material which may be used for the soft cannula 14 is hydrogel material, best known as the primary material in soft contact lenses. Hydrogel material is ideal since it is rigid when dry, making a hydrogel cannula easily insertable. Inside body tissue, the hydrogel cannula would lose its rigidity, and become soft and supple and would therefore be more comfortable. Additionally, insulin absorbtion may be better controlled with a hydrogel cannula since the entire length of the hydrogel cannula could act as a depot site for insulin absorbtion. The hydrogel cannula may be fastened to the hub 48 by a solvent, or by being swedged or heat sealed.

The septum layers 52,54 may be sealably bonded to the upper portion of the hub 48 so as to cover or seal the chamber 50, although this bonding operation is optional. As indicated previously, these layers are typically sized somewhat larger than the openings into which they are inserted, thereby ensuring that appropriate compressive forces are radially applied thereto. Next, the insertion needle 22 is inserted through the septum layers 52,54 and through the lumen 58 of the cannula 14. Once the insertion needle 22 is in place, a cap 56 is placed over the septum layers 52, 54 thereby applying a shearing compressive force thereto and sealing the chamber 50. This cap 56 may be secured in place either by a snap fit, a sonic seal, a solvent bond, or a cold roll process, thereby ensuring that sufficient compressive forces are applied thereto. Alternatively, the insertion needle 22 may be inserted after the installation of the cap 56.

Finally, the pressure sensitive adhesive layer 32 may be applied to the bottom surface 16 of the base portion 46. Pressure-sensitive adhesive may be applied to both sides of a suitable base film or substrate (in a manner similar to double-sided adhesive tape) which is precut to cover the entire bottom surface 16. One side of the adhesive layer 32 is then pressed into position on the bottom surface 16 of the base portion 46. A protective covering or backing 34 is installed on the other side of the adhesive layer 32 to protect the exposed side of the adhesive until such time as the injection set 10 is ready for use.

As indicated previously, the pressure-sensitive adhesive 32 preferably includes an appropriate anti-microbial substance that helps prevent infection and inflammation that may occur at the puncture site. This anti-microbial substance may be coated onto the side of the adhesive layer 32 away from the base portion 46 by suspending a determined percentage of anti-microbial substance in an isopropyl alcohol solution. This solution may then be coated onto the adhesive in the adhesive layer 32 on the side away from the base portion 46, which side will be coming into contact with the epidermis upon installation of the injection set 10. The alcohol may then be driven off by a heat cure normally used during the adhesive coating process.

Referring next to Figure 6, the injection set is shown with the protective backing 34 partially pulled away. The backing 34 may be cut radially so when the pull tab 36 is pulled away from the pressure-sensitive adhesive, the backing 34 peels off of the bottom surface 16 in a spiral motion that does not disturb the protruding soft cannula 14 or the insertion needle 22. This helps ensure that the protruding cannula 14 and the insertion needle 22 are not accidentally bumped or otherwise improperly touched prior to insertion. A needle guard 82 made of clear PVC and installed over the soft cannula 14 and the insertion needle 22 is also helpful in this respect. Figure 6 also shows an alternative embodiment for a handle 80 on the insertion needle 22, essentially in the form of a finger tab which is securely fixed to the end of the insertion needle 22.

An alternative arrangement to the septum layers 52,54 is shown in Figures 7A and 7B. A three layer septum arrangement is illustrated between the central hub 48 and the cap 56. A layer of silicone gel such as Dow Corning Q7-2218 silicone gel is sandwiched between a first layer of vulcanised silicone 152 and a second layer of vulcanised silicon 154 to produce a laminated segment. The laminated segment would preferably be constructed prior to installation between the central hub 48 and the cap 56. The silicone gel 84 is viscous enough to remain sandwiched between the layers of vulcanised silicone 152,154. The three layer segment of septum material may be cut to size by the use of a laser or other means.

In Figure 7A, the insertion needle 22 is shown extending through the layers of vulcanised silicone 152,154 and the silicone gel 84 contained therebetween. Following removal of the insertion needle 22, residual openings 172,174 will exist in the layers of vulcanised silicone 152,154, as shown in Figure 7B. The silicone gel 84 is too viscous to seep through the residual openings 172,174, and will thereby assure a complete and total seal of the residual openings 172,174.

Two additional alternative embodiments of the present invention are illustrated in Figures 8 and 9. These embodiments are for use with standard infusion sets, the embodiment shown in Figure 8 being used with a standard infusion set having a needle with a substantial bend, and the embodiment shown in Figure 9 being used with a standard infusion set having a straight needle.

Referring first to Figure 8, a soft cannula button infusion 210 is illustrated which has a central hub 248 mounted in a domed base 246. The central hub 248 is substantially similar to the central hub 48 of the primary embodiment of Figures 2 and 4, but the central hub 248 lacks an opening for a delivery tube. Two septum layers 252, 254 are mounted in the central hub 248, and are secured by a cap 256 in a manner substantially similar to the primary embodiment of the present invention. Again, a soft cannula 214 is secured to the central hub 248 and extends through the domed base 246. A layer of pressure-sensitive adhesive 232 is used to secure the soft cannula button infuser 210 to the epidermis 38.

The installation of the soft cannula button infuser 210 is identical to the installation of the embodiment of the present invention shown in Figure 2. An insertion needle 22 would be inserted through the septum layers 252,254 and through the lumen of the soft cannula 214. The soft cannula button infuser 210 could then be installed into the skin of the user, and the insertion needle 22 withdrawn as described in conjunction with the embodiment of Figures 2 and 4.

A standard infusion set 286 fed by a delivery tube 288 and having a 27 gauge angle needle 290 is used to supply fluid to the soft cannula button infuser 210. When the angled needle 290 is inserted through the septum layers 252,254, it may be seen that the delivery tube 288 will extend from the soft cannula button infuser 210 in a position substantially parallel to the skin.

The soft cannula button infuser 210 shown in Figure 8 possesses a substantial advantage over the embodiment of the present invention shown in Figures 2 and 4 in that the soft cannula button infuser 210 facilitates a quick disconnect from the source of fluid by merely removing the angled needle 290 from the device, whereupon the septum layers 252,254 will immediately seal. Such a quick disconnect affords the patient the flexibility of taking a shower, bath or swim without necessitating the removal of the injection set and reintroduction of a new injection set following the completion of the activity.

A second alternative embodiment having the same advantages of the embodiment illustrated in Figure 8 is shown in Figure 9. A soft cannula side port button infuser 310 has a central hub 348 mounted in a domed base 346 which is attached to the epidermis 38 by the use of a layer of pressure-sensitive adhesive 332. The central hub 348 has a soft cannula 314 connected thereto in a matter substantially similar to that described above in conjunction with Figure 8, as well as the primary embodiment of the present invention shown in Figures 2 and 4. Two septum layers 352, 354 are mounted in the central hub 348 and held in place by a cap 356, as discussed above. The introduction of the soft cannula side port button infuser 310 to the patient is exactly as discussed above in conjunction with Figure 8.

The soft cannula side port button infuser 310 of Figure 9 differs from the device shown in Figure 8 in that it has a side port for admitting the fluid to the central hub 348. Two additional septum layers 392,394 are mounted in the central hub 348 at a location in a side of the central hub 348 which is open. The septum layers 392,294 are secured in the location in the side of the central hub 348 by an additional cap 396.

A standard infusion set 386 fed by a delivery tube 388 and having a straight 27 gauge needle may be used to supply the soft cannula side port button infuser 310. The straight needle 390 is inserted through the septum layers 392,394, so that the needle 390 is in communication with the interior of the central hub 348. It should be noted that the domed base 346 does not extend completely around the central hub 348, with an opening being left in the domed base 346 around the cap 349. It may be appreciated that the soft cannula side port button infuser 310 illustrated in Figure 9 has the lowest profile of any of the devices discussed in this specification, since the infusion set 386 is connected to the device at the side thereof. Therefore, it may be seen that the soft cannula side port button infusion 310 illustrated in Figure 9 possesses the same advantage of the device illustrated in Figure 8, namely that it facilitates convenient disconnection and reconnection of the device to a source of fluid.

Because of the simplicity of the injection set 10 described above, it can be used as a disposable device which may be conveniently used by the patient without the need of medical assistance from a nurse or doctor. After appropriate usage, it may be discarded. A typical use of the injection set 10 will be by diabetics who receive a controlled does of insulin from an external insulin infusion pump. The patient need only load the external infusion pump with the source of fluid and connect it to the injection set 10, prime the injection set 10, and then insert the injection set 10 at an appropriate skin location. This entire operation may be completed in a very short time period (e.g. less than 60 seconds). With the miniaturised external infusion pumps now available, and with an injection set as described herein, it is unlikely that anyone other than the patient will know or be able to determine that an infusion pump and injection set 10 are being used.

## Claims

1. A subcutaneous injection set (10) comprising a housing, a hollow needle (14) protruding from the housing and means (18,50) for connecting a source of fluid to the needle (14); the housing including a hub (48) having a chamber (50) therein, and self-sealing means (52, 54) for enclosing the chamber (50) at the top of the hub (48); the needle (14) comprising a soft or flexible cannula having a lumen (58) which is in fluid communication with the chamber (50) within the housing, and an insertion needle (22) for inserting the protruding portion of the cannula (14) into a subcutaneous layer (42) of the patient, the insertion needle (22) passing removably through the self-sealing means (52, 54) at the top of the hub, through the chamber (50) and through the lumen (58) of the soft cannula with a tight fit, the insertion needle (22) having a sharpened tip extending beyond the length of the soft cannula when the insertion needle (22) is fully inserted through the housing, the self-sealing means (52, 54) sealing the top of the hub (48) when the insertion needle (22) is withdrawn therefrom, characterised in that the housing comprises a holding pad (12) with a substantially flat base (16) for placement on the skin (38) of a patient at an injection site, means (32) for removably securing the holding pad (12) to the skin of a patient; and in which the hub (48) is mounted on top of the holding pad (12) and the soft cannula extends from the chamber (50) through the hub (48) and the holding pad (12).

2. An injection set as claimed in Claim 1 characterised in that the self-sealing means comprises a septum (52) made from a pierceable resilient resealable substance.

3. An injection set as claimed in Claim 2 characterised by compression means (54) for subjecting the self-sealing septum affixed to the upper side of the housing to constant compressive forces, whereby any hole made in the septum (52) by the insertion needle (22) is tightly closed by compressive forces, once the insertion needle (22) is removed.

4. An injection set as claimed in Claim 3 characterised in that the compressive forces include both radial compressive forces and shearing compressive forces acting on the septum.

5. An injection set as claimed in Claim 3 or Claim 4 characterised in that the compression means includes a cap (54) which is affixed to the upper side of the housing (12) over the septum (52) in a way which exerts a compressive force on the septum (52), the cap having a small hole through which the insertion needle (22) may be inserted.

6. An injection set as claimed in any preceding claim characterised by a fluid inlet to the chamber (50) through a side of the housing adjacent to and between the base (16) and upper side of the housing (12).

7. An injection set as claimed in Claim 6 characterised in that the fluid inlet includes a flexible tube (18) in fluid communication with the chamber (50) within the housing (12), the flexible tube (18) extending from the side wall of the housing (12) at an angle which is substantially parallel to the flat base (16).

8. An injection set as claimed in Claim 6 characterised in that the fluid inlet is closed by a second self-sealing septum (392) through which an infusion needle (390) may be removably inserted.

9. An injection set as claimed in any preceding Claim characterised in that the means for detachably securing the flat base (16) of the housing (12) to the skin (38) of the patient, comprises an adhesive layer (32) placed on the flat base (16).

10. An injection set as claimed in any preceding claim characterised by means for priming the injection set when the chamber (50) is connected to a source of fluid prior to insertion of the insertion needle (22) and the soft cannula (14) into the skin (38) of a patient, the priming means comprising a passage (62,64) between the tip (24) of the insertion needle (22) and a section of the insertion needle (22) within the chamber, the passage (62,64) being in fluid communication with the chamber, whereby all air or other gases within the fluid chamber may escape through the passage (62,64) when the tip (24) of the insertion needle (22) is held so as to point upwards with respect to the skin of the patient as fluid is introduced under pressure into the chamber.

**Revendications**

1. Appareil d'injection sous-cutanée (10) comprenant un logement, une aiguille creuse (14) qui est saillante sur le logement et des organes (18, 50) de raccord d'une source de fluide à l'aiguille (14) ; le logement comportant un cylindre (48) renfermant une chambre (50) et des éléments auto-obturateurs (52, 54) destinés à fermer la chambre (50) au sommet du cylindre (48) ; l'aiguille (14) comprenant une canule souple ou flexible comportant un passage (58) qui communique avec la chambre (50) située dans le logement pour le passage de fluide, ainsi qu'une aiguille d'insertion (22) destinée à faire pénétrer la partie saillante de la canule (14) dans une couche sous-cutanée (42) du patient, l'aiguille d'insertion (22) passant de manière amovible à travers les éléments auto-obturateurs (52, 54) situés au sommet du cylindre, par la chambre (50) et dans le passage (58) de la canule souple dans laquelle elle est enserrée étroitement, l'aiguille d'insertion (22) comportant une pointe éffilée qui se prolonge au-delà de la canule souple lorsque l'aiguille d'insertion (22) est entièrement introduite dans le logement, les éléments auto-obturateurs (52, 54) assurant l'étanchéité au sommet du cylindre (48) lorsque l'aiguille d'insertion (22) en est retirée, caractérisé en ce que le logement comporte un tampon de retenue (12) ayant une base sensiblement plane (16) destinée à être placée sur la peau (38) d'un patient au lieu d'injection, un moyen (32) de fixation amovible du tampon de retenue (12) à la peau d'un patient ; et en ce que le cylindre (48) est monté au sommet du tampon de retenue (12) et la canule souple part de la chambre (50) et passe par le cylindre (48) et le tampon de retenue (12).

2. Appareil d'injection selon la revendication 1, caractérisé en ce que les éléments auto-obturateurs consistent en une cloison (52) faite d'une substance élastique pouvant être percée et assurant le rétablissement de l'étanchéité.

3. Appareil d'injection selon la revendication 2, caractérisé par des éléments de compression (54) destinés à soumettre la cloison auto-obturante fixée au côté supérieur du logement à des forces constantes de compression, de manière que tout trou fait dans la cloison (52) par l'aiguille d'insertion (22) soit fermé de manière étanche par les forces de compression après que l'aiguille d'insertion (22) a été enlevée.

4. Appareil d'injection selon la revendication 3, caractérisé en ce que les forces de compression comprennent aussi bien des forces de compression radiale que des forces de compression agissant en cisaillement, qui s'exercent sur la cloison.

5. Appareil d'injection selon la revendication 3 ou 4, caractérisé en ce que les éléments de compression comprennent un capuchon (54) qui est fixé au côté supérieur du logement (12), au-dessus de la cloison (52), de manière à exercer une force de compression sur la cloison (52), le capuchon comportant un petit trou par lequel l'aiguille d'insertion (22) peut être introduite.

6. Appareil d'injection selon l'une quelconque des revendications précédentes, caractérisé par une entrée de fluide dans la chambre (50) par un côté du logement qui est voisin de et situé entre la base (16) et le côté supérieur du logement (12).

7. Appareil d'injection selon la revendication 6, caractérisé en ce que l'entrée de fluide comprend un tube flexible (18) qui communique avec la chambre (50) située dans le logement (12) pour le passage de fluide, le tube flexible (18) partant de la paroi latérale du logement (12) sous un angle qui est sensiblement parallèle à la base plane (16).

8. Appareil d'injection selon la revendication 6, caractérisé en ce que l'entrée de fluide est fermée par une seconde cloison auto-obturante (392) à travers laquelle une aiguille d'arrivée (390) peut être introduite de manière amovible.

9. Appareil d'injection selon l'une quelconque des revendications précédentes, caractérisé en ce que le moyen de fixation amovible de la base plane (16) du logement (12) à la peau (38) du patient comprend une couche d'adhésif (32) placée sur la base plane (16).

10. Appareil d'injection selon l'une quelconque des revendications précédentes, caractérisé par des moyens d'amorçage de l'appareil d'injection lorsque la chambre (50) est raccordée à une source de fluide avant l'introduction de l'aiguille d'insertion (22) et de la canule souple (14) dans la peau (38) d'un patient, les moyens d'amorçage comprenant un passage (62, 64) entre la pointe (24) de l'aiguille d'insertion (22) et une section de l'aiguille d'insertion (22) qui est située à l'intérieur de la chambre, le passage (62, 64) communiquant avec la chambre pour le passage de fluide, de manière que tout air ou autres gaz situés à l'intérieur de la chambre à fluide puissent s'échapper par le passage (62, 64) lorsque la pointe (24) de l'aiguille d'insertion (22) est tenue de manière à être orientée vers le haut par rapport à la peau du patient au moment auquel du fluide est introduit sous pression dans la chambre.

**Patentansprüche**

1. Subkutanes Injektionsbesteck (10) mit einem Gehäuse, einer aus dem Gehäuse vorstehenden Hohlnadel (14) und Mitteln (18, 50) zur Verbindung einer Flüssigkeitsquelle mit der Nadel (14), wobei das Gehäuse ein Zwischenstück (48) mit einer Kammer (50) und selbstdichtende Mittel (52, 54) zum Abschluß der Kammer (50) am oberen Ende des Zwischenstücks (48) aufweist und die Nadel (14) eine weiche oder flexible Kanüle mit einem Lumen (58), das in Flüssigkeitsverbindung mit der Kammer (50) innerhalb des Gehäuses steht, und eine Injektionsnadel (22) zur Einführung des vorstehenden Abschnitts der Kanüle (14) in eine subkutane Schicht (42) des Patienten umfaßt, wobei die Injektionsnadel (22) verschieblich durch die selbst-dichtenden Mittel (52, 54) am oberen Ende des Zwischenstükkes, durch die Kammer (50) und mit einem festen Sitz durch das Lumen (58) der weichen Kanüle verläuft und eine scharfe Spitze aufweist, die über die Länge der weichen Kanüle hinausragt, wenn die Injektionsnadel (22) vollständig durch das Gehäuse hindurchgeführt ist, wobei die selbst-dichtenden Mittel (52, 54) das obere Ende des Zwischenstücks (48) abdichten, wenn die Injektionsnadel (22) aus diesem wieder herausgezogen wird, **dadurch gekennzeichnet,** daß das Gehäuse ein Haltekissen (12) mit einer im wesentlichen flachen Grundfläche (16) zur Auflage auf die Haut (38) eines Patienten an der Injektionsstelle und Mittel (32) zur lösbaren Befestigung des Haltekissens (12) auf der Haut eines Patienten umfaßt, daß das Zwischenstück (48) oben am Haltekissen (12) angebracht ist und daß die weiche Kanüle von der Kammer (50) durch das Zwischenstück (48) und das Haltekissen (12) hindurch verläuft.

2. Injektionsbesteck nach Anspruch 1, dadurch gekennzeichnet, daß die selbst-dichtenden Mittel ein aus einer durchstoßbaren, federnden, wieder abdichtenden Substanz hergestelltes Septum (52) umfassen.

3. Injektionsbesteck nach Anspruch 2, gekennzeichnet durch Druckmittel (54) zum Aufbringen von konstanten Druckkräften auf das an der Oberseite des Gehäuses befestigte, selbstdichtende Septum, wobei jedes durch die Injektionsnadel (22) im Septum (52) erzeugte Loch durch Druckkräfte wieder dicht verschlossen wird, wenn die Injektionsnadel (22) entfernt wird.

4. Injektionsbesteck nach Anspruch 3, dadurch gekennzeichnet, daß die auf das Septum wirkenden Druckkräfte sowohl Radial-, wie auch Scher-Druckkräfte umfassen.

5. Injektionsbesteck nach Anspruch 3 oder 4, dadurch gekennzeichnet, daß die Druckmittel eine Kappe (56) umfassen, die an der Oberseite des Gehäuses (12) über dem Septum (52) derart befestigt ist, daß eine Druckkraft auf das Septum (52) ausgeübt wird, wobei die Kappe ein kleines Loch aufweist, durch das die Injektionsnadel (22) einführbar ist.

6. Injektionsbesteck nach einem der vorhergehenden Ansprüche, gekennzeichnet durch einen Flüssigkeitseinlaß zu der Kammer (50) durch eine Gehäuseseite in der Nähe der Grundfläche (16) sowie zwischen dieser und der oberen Seite des Gehäuses (12).

7. Injektionsbesteck nach Anspruch 6, dadurch gekennzeichnet, daß der Flüssigkeitseinlaß ein flexibles Rohr (18) umfaßt, das in Strömungsverbindung mit der Kammer (50) im Gehäuse (12) steht, wobei das flexible Rohr (18) von der Seitenwand des Gehäuses (12) aus in einem Winkel verläuft, der im wesentlichen parallel zur flachen Grundfläche (16) liegt.

8. Injektionsbesteck nach Anspruch 6, dadurch gekennzeichnet, daß der Flüssigkeitseinlaß durch ein zweites selbst-dichtendes Septum (392) verschlossen ist, durch das eine Infusionsnadel (390) herausnehmbar eingeführt werden kann.

9. Injektionsbesteck nach einem der vorhergehenden Ansprüche, dadurch gekennzeichnet, daß die Mittel zur lösbaren Befestigung der flachen Grundfläche (16) des Gehäuses (12) auf der Haut (38) des Patienten eine auf der flachen Grundfläche (16) angeordnete Haftschicht (32) umfassen.

10. Injektionsbesteck nach einem der vorhergehenden Ansprüche, gekennzeichnet durch Mittel zur Vorbereitung des Injektionsbesteckes, wenn die Kammer (50) vor dem Einführen der Injektionsnadel (22) und der weichen Kanüle (14) in die Haut (38) eines Patienten an eine Flüssigkeitsquelle angeschlossen ist, wobei die Vorbereitungsmittel einen in Strömungsverbindung mit der Kammer stehenden Durchlaß (62, 64) zwischen der Spitze (24) und einem Abschnitt der Injektionsnadel (22) innerhalb der Kammer umfassen, wodurch sämtliche Luft oder andere Gase innerhalb der Flüssigkeitskammer durch den Durchlaß (62, 64) entweichen können, wenn die Spitze (24) der Injektionsnadel (22) in bezug auf die Haut des Patienten über dieser gehalten wird, sobald Flüssigkeit unter Druck in die Kammer eingelassen wird.

FIG. 1

FIG. 2

FIG. 3A          FIG. 3B          FIG. 3C

_FIG. 6_

_FIG. 4_

FROM INFUSION PUMP

_FIG. 5A_

_FIG. 5B_

Fig. 7A

Fig. 7B

Fig. 8

Fig. 9